# EUROPEAN PATENT APPLICATION

(11) **EP 4 818 732 A1**
(43) Date of publication of application: **07.10.2026**
(21) Application number: 23962925.6
(22) Date of filing: 29.12.2023
(51) Int. Cl.: G01R 33/48, A61B 5/055

(54) **METHODS AND SYSTEM FOR RECONSTRUCTING FOUR-DIMENSIONAL MAGNETIC RESONANCE IMAGE**

(71) Applicant: Shanghai United Imaging Healthcare Co., Ltd., Shanghai 201807 (CN)
(72) Inventor: FANG, Xiangbin, Shanghai 201807 (CN); LIAO, Can, Shanghai 201807 (CN)
(74) Representative: Wang, Bo
(86) International application number: PCT/CN2023/143613
(87) International publication number: WO 2025/138251

(57) **Abstract**

The embodiments of the present description provide methods and system for constructing 4D MR images. A method comprises: determining a data acquisiticon mode of each phase on the basis of a real-time motion signal of an object; acquiring original data of the object according to the dataacquisition mode, classifying the original data to obtain data of multiple categories, wherein data of each category corresponds to a different phase, and performing image reconstruction on the data of the multiple categories to obtain a four-dimensional magnetic resonance image.

## Description

### TECHNICAL FIELD

The present disclosure relates to the field of imaging technology, and in particular, to a method and system for reconstructing four-dimensional (4D) magnetic resonance (MR) image.

### BACKGROUND

In the field of radiotherapy, imaging has become a key technology for improving therapeutic effects, and the quality of images often significantly affects the treatment accuracy of radiotherapy beams. However, due to the presence of respiratory motion and heartbeat in patients during treatment, static images often cannot be used as a reference for treatment. 4D MR imaging (briefly referred as 4D MRI), due to its outstanding soft tissue contrast and lack of ionizing radiation, makes the optimization of radiotherapy treatment plans more efficient, improves the accuracy of radiotherapy, and provides superior imaging references for the field of radiotherapy.

### SUMMARY

One embodiment of the present disclosure provides a method for 4D MR image reconstruction, comprising: determining a data acquisition mode of each phase on the basis of a real-time motion signal of an object; acquiring original data of the object according to the data acquisition mode; classifying the original data to obtain data of multiple categories, wherein data of each category corresponds to a different phase; and performing image reconstruction on the data of multiple categories to obtain the four-dimensional magnetic resonance image.

One embodiment of the present disclosure further provides a method for 4D MR image reconstruction, comprising: determining full k-space data for each phase on the basis of acquired original data; for each phase, performing image reconstruction on the basis of the full k-space data to obtain a corresponding reconstructed image; and combining the reconstructed images of each phase to obtain a 4D MR image, wherein determining the full k-space data for each phase comprises: for each undersampled target phase, supplementing the unacquired data in the target phase on the basis of at least one of sampled data from a stable motion phase or sampled data from a candidate phase having more sampled data than the target phase, to obtain the full k-space data for the target phase.

One embodiment of the present disclosure further provides a system for 4D MR image reconstruction, comprising: at least one non-transitory storage medium comprising a set of instructions; and one or more processors in communication with the at least one non-transitory storage medium, wherein, when the instructions are executed, the one or more processors are configured to: determine a data acquisition mode of each phase on the basis of a real-time motion signal of an object; acquire original data of the object according to the data acquisition mode; classify the original data to obtain data of multiple categories, wherein data of each category corresponds to a different phase; and performing image reconstruction on the data of multiple categories to obtain the four-dimensional magnetic resonance image.

One embodiment of the present disclosure further provides a computer-readable non-transitory storage medium, the non-transitory storage medium storing computer instructions, wherein when a computer reads the computer instructions in the non-transitory storage medium, the computer executes the following method: determining a data acquisition mode of each phase on the basis of a real-time motion signal of an object; acquiring original data of the object according to the data acquisition mode; classifying the original data to obtain data of multiple categories, wherein data of each category corresponds to a different phase; and performing image reconstruction on the data of multiple categories to obtain the four-dimensional magnetic resonance image.

### BRIEF DESCRIPTION OF THE DRAWINGS

The present disclosure will be further described by way of exemplary embodiments, which will be described in detail with reference to the accompanying drawings. These embodiments are non-limiting, and in these embodiments, like reference numbers represent like parts throughout the drawings, wherein:
FIG. 1 is a schematic diagram of an exemplary system for 4D MR image reconstruction according to some embodiments of the present disclosure;
FIG. 2 is a schematic diagram of hardware and/or software of an exemplary computing device according to some embodiments of the present disclosure;
FIG. 3 is a schematic module illustration of an exemplary 4D MR image reconstruction apparatus according to some embodiments of the present disclosure;
FIG. 4 is a schematic flowchart of an exemplary 4D MR image reconstruction method of some embodiments of the present disclosure;
FIG. 5A is a schematic diagram of an exemplary phase division of a respiratory signal according to some embodiments of the present disclosure;
FIG. 5B is a schematic diagram of an exemplary phase division of a respiratory signal according to some embodiments of the present disclosure;
FIG. 6 is a schematic diagram of an exemplary phase of a respiratory signal according to some embodiments of the present disclosure;
FIG. 7(a) is a schematic diagram of an exemplary k-space filling for Cartesian acquisition according to some embodiments of the present disclosure;
FIG. 7(b) is a schematic diagram of an exemplary k-space filling for Cartesian acquisition according to some embodiments of the present disclosure;
FIG. 8(a) is a schematic diagram of an exemplary k-space filling for Zig-Zag acquisition according to some embodiments of the present disclosure;
FIG. 8(b) is a schematic diagram of an exemplary k-space filling for Zig-Zag acquisition according to some embodiments of the present disclosure;
FIG. 9(a) is a schematic diagram of an exemplary k-space filling for radial acquisition according to some embodiments of the present disclosure;
FIG. 9(b) is a schematic diagram of an exemplary k-space filling for radial acquisition according to some embodiments of the present disclosure;
FIG. 10(a) is a schematic diagram of an exemplary k-space filling for spiral acquisition according to some embodiments of the present disclosure;
FIG. 10(b) is a schematic diagram of an exemplary k-space filling for spiral acquisition according to some embodiments of the present disclosure;
FIG. 11(a) is a schematic diagram of an exemplary k-space filling for windmill acquisition according to some embodiments of the present disclosure;
FIG. 11(b) is a schematic diagram of an exemplary k-space filling for windmill acquisition according to some embodiments of the present disclosure;
FIG. 12(a) is a schematic diagram of an exemplary k-space filling for radial 3D acquisition according to some embodiments of the present disclosure;
FIG. 12(b) is a schematic diagram of an exemplary k-space filling for radial 3D acquisition according to some embodiments of the present disclosure;
FIG. 13 is a schematic diagram of an exemplary k-space filling for undersampling according to some embodiments of the present disclosure;
FIG. 14(a) is a schematic diagram of an exemplary k-space filling for undersampling according to other embodiments of the present disclosure;
FIG. 14(b) is a schematic diagram of an exemplary k-space filling for undersampling according to other embodiments of the present disclosure; and
FIG. 15 is a schematic diagram of an exemplary k-space filling for fully sampling according to some embodiments of the present disclosure.

### DETAILED DESCRIPTION

In order to more clearly illustrate the technical solutions of the embodiments of the present disclosure, the accompanying drawings required for describing the embodiments are briefly introduced as follows. Obviously, the drawings in the following description are only some examples or embodiments of the present disclosure, and for those of ordinary skill in the art, other similar scenarios can be derived from these drawings without inventive effort. Unless otherwise apparent from the language context or otherwise stated, like reference numbers in the figures represent like structures or operations.

It should be understood that the terms "system", "apparatus", "unit", and/or "module" used herein are used for distinguishing different levels of different components, elements, parts, sections, or assemblies. However, if other words can achieve the same purpose, the words may be replaced by other expressions.

As shown in this disclosure and the claims, unless the context clearly indicates an exception, words such as "a", "an", "one", and/or "the" do not specifically refer to the singular but may also refer to the plural. Generally speaking, the terms "comprising" and "including" only indicate the inclusion of clearly identified steps and/or elements, and these steps and/or elements do not constitute an exclusive listing, and the method or device may also include other steps or elements.

Flowcharts are used in this disclosure to illustrate the operations performed by the system according to the embodiments of the present disclosure. It should be understood that the preceding or following operations are not necessarily performed in exact order. In contrast, the steps may be processed in reverse order or simultaneously. Meanwhile, other operations may also be added to these flows of the operations, or one or more steps may be removed from these flows of the operations.

This disclosure provides systems and methods for non-invasive imaging and/or treatment, for example, for disease diagnosis, treatment, or research purposes. In some embodiments, the system may include a radiation therapy (RT) system, a computed tomography (CT) system, an emission computed tomography (ECT) system, a radiography system, a positron emission tomography (PET) system, a magnetic resonance imaging (MRI) system, etc., or any combination thereof. For illustrative purposes, this disclosure describes systems and methods for 4D MR image reconstruction. In this disclosure, the term "image" may refer to a 2D image, a 3D image, or a 4D image. In some embodiments, the term "image" may refer to an image of a region of a patient (e.g., a region of interest (ROI)).

As mentioned above, 4D MRI images provide superior image reference for the field of radiation therapy due to their outstanding soft tissue contrast and non-ionizing radiation characteristics. However, due to the specific nature of magnetic resonance imaging technology, the acquisition speed of actual image data is limited, often resulting in issues such as motion artifacts and low temporal resolution.

In some embodiments, on the basis of a respiratory signal, the acquired signal data can be assigned with respiratory amplitude information at the time of acquisition, assisting in combining k-space data under the same respiratory state to form complete and reconstructable magnetic resonance original data, and an inverse Fourier transform is then performed on the combined data to obtain a 4D MRI image. However, this method suffers from long scan times and large data redundancy.

Embodiments of this disclosure provide a system and method for 4D MR image reconstruction. On the basis of real-time motion information of the object, when the object is in a stable motion state, more data is acquired to keep the high-frequency regions of k-space (e.g., edge positions) fully filled. When the object is in a state with large motion amplitude fluctuations, data from the central region of k-space is acquired while high-frequency k-space data is not acquired, maintaining the capture of image contrast information, thereby enabling the image to have high temporal resolution. Through this method, image acquisition time can be further reduced and therefore scanning efficiency can be improved without degrading or even while enhancing image quality.

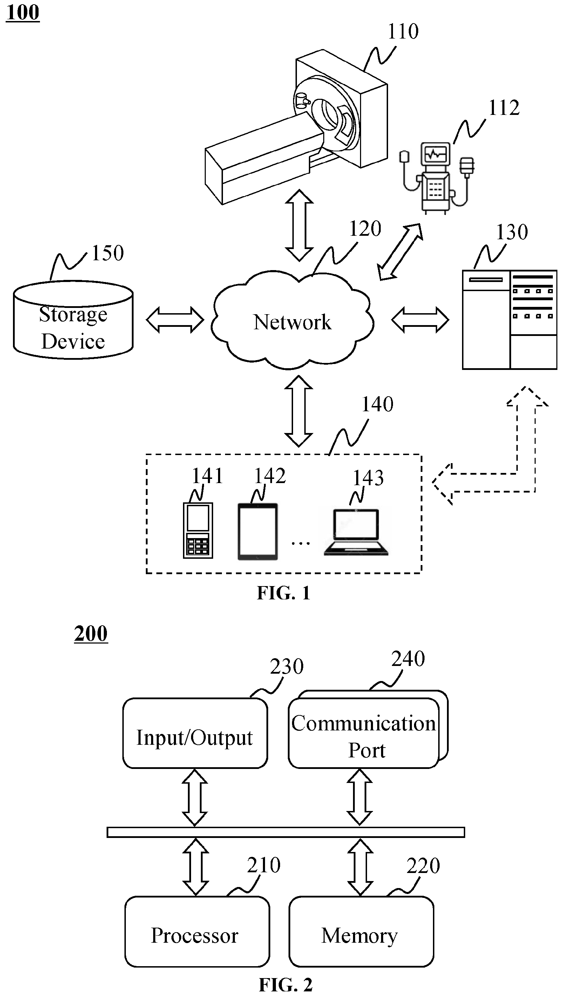

FIG. 1 is a schematic diagram of an exemplary 4D MR image reconstruction system according to some embodiments of the present disclosure.

As shown in FIG. 1, the magnetic resonance image reconstruction system 100 may include an imaging device 110, a motion signal acquisition device 112, a network 120, a processing device 130, a terminal device 140, and a storage device 150. In some embodiments, various components in the magnetic resonance image reconstruction system 100 may be connected to each other via the network 120 or connected to each other directly without the network 120. For example, the imaging device 110 and the terminal device 140 may be connected via the network 120. For another example, the imaging device 110 and the processing device 130 may be connected to each other via the network 120 or directly. For another example, the processing device 130may be connected to the terminal device 140 via the network 120 or directly to the terminal device 140.

The imaging device 110 may scan an object within a detection area or scan area to obtain scan data of the object (e.g., original data, reconstructed images, etc.). For example, the imaging device 110 may acquire original data of the object according to a determined data acquisition mode. The object may be biological or non-biological. By way of example only, the object may include a patient, an artificial object (e.g., an artificial phantom), etc. For another example, the object may include at least one of a specific part, organ, or tissue of a patient (e.g., head, ear, nose, oral cavity, neck, chest, abdomen, liver, gallbladder, pancreas, spleen, kidney, spine, heart, tumor tissue or the like).

In some embodiments, the imaging device 110 may include one or a combination of X-ray equipment, Computed Tomography (CT) equipment, ultrasound imaging components, fluoroscopy imaging components, Magnetic Resonance Imaging (MRI) equipment, Single Photon Emission Computed Tomography (SPECT) equipment, Positron Emission Tomography (PET) equipment, etc.

In some embodiments, the imaging device 110 may be an MRI device. An MRI device uses a strong magnetic field, gradient magnetic fields, and radio waves to generate magnetic resonance images (e.g., two-dimensional magnetic resonance images, three-dimensional magnetic resonance images, 4D MR images, etc.) of the object to be scanned. In some embodiments, the MRI device may include a magnet assembly, a gradient coil assembly, and a radio frequency (RF) coil assembly. The magnet assembly may generate a first magnetic field (also referred to as the main magnetic field) for polarizing the object. The gradient coil assembly may be used to generate a second magnetic field (also referred to as the gradient magnetic field).

In some embodiments, the gradient coil assembly may generate one or more magnetic field gradient pulses superimposed on the main magnetic field in the X direction (Gx), Y direction (Gy), and Z direction (Gz) to encode spatial information of the object. In some embodiments, the X direction may be designated as the frequency encoding direction, and the Y direction may be designated as the phase encoding direction. In some embodiments, Gx may be used for frequency encoding or signal readout, and is often referred to as the frequency encoding or readout (RO) gradient. In some embodiments, Gy may be used for phase encoding, and is often referred to as the phase encoding (PE) gradient. In some embodiments, Gz may be used for slice selection to acquire two-dimensional k-space data, and is often referred to as the slice selection (SS) gradient. In some embodiments, Gz may be used for phase encoding to acquire three-dimensional k-space data.

In some embodiments, the RF coil may include one or more transmit coils and/or one or more receive coils. The RF transmit coil may transmit RF pulses to the object. Under the synergistic effect of the main magnetic field/gradient magnetic fields and RF pulses, magnetic resonance signals related to the object may be generated according to a pulse sequence. The RF receive coil may acquire magnetic resonance signals from the object according to the pulse sequence. Magnetic resonance signals may also be referred to as echo signals. A transform operation (e.g., Fourier transform) may be used to process the magnetic resonance signals to fill k-space and obtain k-space data, thereby generating a magnetic resonance image of the object.

A pulse sequence may be defined by imaging parameters and the temporal arrangement of the imaging parameters. For example, a pulse sequence may be defined by one or more time-dependent parameters (e.g., acquisition time (TA), echo time (TE), etc.). Exemplary pulse sequences may include spin echo sequences, gradient echo sequences, diffusion sequences, inversion recovery sequences, etc., or any combination thereof. For example, spin echo sequences may include Fast Spin Echo (FSE), Turbo Spin Echo (TSE), Rapid Acquisition with Relaxation Enhancement (RARE), Half-Fourier Acquisition Single-shot Turbo Spin Echo (HASTE), Turbo Gradient Spin Echo (TGSE), etc., or any combination thereof. In some embodiments, the pulse sequence may include a diffusion sequence. A diffusion sequence may include at least one diffusion block associated with one or more parameters of a diffusion gradient and at least one imaging block associated with one or more scan parameters (e.g., parameters associated with one or more encoding gradients applied in the scan). In some embodiments, the pulse sequence may include a single-shot pulse sequence (e.g., single-shot FSE, single-shot EPI, etc.), a multi-shot pulse sequence (e.g., multi-shot FSE, multi-shot EPI, etc.). In some embodiments, for a single-shot pulse sequence (e.g., single-shot FSE, single-shot EPI, etc.), an RF pulse may be applied once within one TR (repetition time, TR), and one or more echo signals may be sampled within the TR. For a multi-shot pulse sequence (e.g., multi-shot FSE, multi-shot EPI, etc.), RF pulses may be applied multiple times within one TR, and one or more echo signals may be sampled during each shot or excitation.

The motion signal acquisition device 112 may be used to acquire real-time motion signals (e.g., respiratory signals, heartbeat signals, etc.) of the object. In some embodiments, the motion signal acquisition device 112 may include a respiratory signal acquisition device or a heartbeat signal acquisition device. In some embodiments, the motion signal acquisition device 112 may include a contact-type motion signal acquisition device and/or a non-contact-type motion signal acquisition device. The contact-type motion signal acquisition device may calculate motion signals via an ECG (electrocardiogram) chest lead or acquire motion signals via a carbon dioxide sensor. The non-contact-type motion signal acquisition device may acquire motion signals via bio-radar, a sensor-based special table pad, optical signal acquisition, or a combination thereof. In some embodiments, the motion signal acquisition device 112 may acquire real-time motion signals in multiple ways. Acquisition methods may include real-time image-based feature recognition technology, optical body surface imaging technology, infrared marker technology, respiratory strap technology, or any combination thereof.

In some embodiments, the motion signal acquisition device 112 may also perform a process (for example, filtering, noise reduction, smoothing, etc.) on the acquired real-time motion signals.

In some embodiments, the motion signal acquired by the motion signal acquisition device 112 may be a motion signal curve with time as the horizontal coordinate and amplitude as the vertical coordinate. In some embodiments, the real-time motion signal acquired by the motion signal acquisition device 112 may include one or more signal cycles. In some embodiments, the motion signal acquisition device 112 may acquire real-time motion signals of the object before, during, and after the scan. In some embodiments, the motion signal acquisition device 112 may acquire real-time motion signals synchronously during the imaging process.

In some embodiments, the motion signal acquisition device 112 may be a part of the imaging device 110, or a device independent of the imaging device 110.

The network 120 may include any suitable network capable of facilitating the exchange of information and/or data for the magnetic resonance image reconstruction system 100. In some embodiments, one or more components of the magnetic resonance image reconstruction system 100 (e.g., the imaging device 110, the motion signal acquisition device 112, the processing device 130, the terminal device 140, and the storage device 150) may exchange information and/or data with one or more components of the magnetic resonance image reconstruction system 100 via the network 120. For example, the processing device 130 may obtain real-time motion signals of the object from the motion signal acquisition device 112 via the network 120. In some embodiments, the network 120 may include a public network (e.g., the Internet), a private network (e.g., a local area network (LAN), a wide area network (WAN)), a wired network (e.g., Ethernet), a wireless network (e.g., an 802.11 network, a wireless Wi-Fi network), a cellular network (e.g., a Long Term Evolution (LTE) network), a frame relay network, a virtual private network (VPN), a satellite network, a telephone network, a router, a hub, a server computer, or a combination of one or more thereof. For example, the network 120 may include a wired network, an optical fiber network, a telecommunications network, a local area network, a wireless local area network (WLAN), a metropolitan area network (MAN), a Public Switched Telephone Network (PSTN), a Bluetooth^{™} network, a ZigBee^{™} network, a Near Field Communication (NFC) network, or a combination of one or more thereof. In some embodiments, the network 120 may include one or more network access points. For example, the network 120 may include wired and/or wireless network access points, such as base stations and/or Internet exchange points, through which one or more components of the magnetic resonance image reconstruction system 100 may connect to the network 120 to exchange data and/or information.

The processing device 130 may process data and/or information obtained from the imaging device 110, the motion signal acquisition device 112, the terminal device 140, the storage device 150, or other components of the magnetic resonance image reconstruction system 100. For example, the processing device 130 may obtain real-time motion signals of the object from the motion signal acquisition device 112, and determine a data acquisition mode of each phase on the basis of the real-time motion signals of the object. In some embodiments, the processing device 130 may be local or remote. For example, the processing device 130 may access information and/or data from the imaging device 110, the motion signal acquisition device 112, the terminal device 140, and/or the storage device 150 via the network 120.

In some embodiments, the processing device 130 and the imaging device 110 may be integrated into one unit. In some embodiments, the processing device 130 and the imaging device 110 may be directly or indirectly connected, working in conjunction to implement the methods and/or functions described in this disclosure.

In some embodiments, the processing device 130 and the motion signal acquisition device 112 may be integrated into one unit. In some embodiments, the processing device 130 and the motion signal acquisition device 112 may be directly or indirectly connected, working in conjunction to implement the methods and/or functions described in this disclosure. In some embodiments, the imaging device 110, the motion signal acquisition device 112, and the processing device 130 may be integrated into one unit. In some embodiments, the imaging device 110, the motion signal acquisition device 112, and the processing device 130 may be directly or indirectly connected, working in conjunction to implement the methods and/or functions described in this disclosure.

In some embodiments, the processing device 130 may include an input device and/or an output device. Interaction with a user (e.g., displaying real-time motion signals, displaying 4D MR images, etc.) may be achieved via the input device and/or the output device. In some embodiments, the input device and/or the output device may include a touch screen, a keyboard, a mouse, a microphone, or any combination thereof.

The terminal device 140 may connect and/or communicate with the imaging device 110, the motion signal acquisition device 112, the processing device 130, and/or the storage device 150. For example, the terminal device 140 may obtain the reconstructed 4D MR image from the processing device 130 and display it. As another example, the terminal device 140 may obtain the data acquisition mode determined on the basis of the real-time motion signal from the processing device 130 and display it. In some embodiments, the terminal device 140 may include a mobile device 141, a tablet computer 142, a laptop computer 143, or any combination thereof. In some embodiments, the terminal device 140 (or all or a part of its functions) may be integrated into the processing device 130.

The storage device 150 may store data, instructions, and/or any other information. In some embodiments, the storage device 150 may store data obtained from the imaging device 110, the motion signal acquisition device 112, and/or the processing device 130 (e.g., real-time motion signals, data acquisition modes, tagged original data, 4D MR images, etc.). In some embodiments, the storage device 150 may store computer instructions for implementing the 4D MR image reconstruction method, etc.

In some embodiments, the storage device 150 may include one or more storage components, each of which may be an independent device or part of another device. In some embodiments, the storage device 150 may include a random access memory (RAM), a read-only memory (ROM), a mass storage device, a removable storage device, a volatile read/write memory, or any combination thereof. Exemplarily, the mass storage device may include a magnetic disk, an optical disc, a solid-state disk, etc. RAM may include dynamic RAM (DRAM), double data rate synchronous dynamic RAM (DDR SDRAM), static RAM (SRAM), thyristor RAM (T-RAM), and zero-capacitor RAM (Z-RAM), among others. ROM may include mask ROM (MROM), programmable ROM (PROM), erasable programmable ROM (EPROM), electrically erasable programmable ROM (EEPROM), compact disc ROM (CD-ROM), and digital versatile disc ROM, among others. In some embodiments, the storage device 150 may be implemented on a cloud platform.

It should be noted that the magnetic resonance image reconstruction system 100 is provided for illustrative purposes only and is not intended to limit the scope of the present disclosure. For those of ordinary skill in the art, various modifications or variations may be made on the basis of the description of the present disclosure. For example, the magnetic resonance image reconstruction system 100 may include one or more medical devices. In some embodiments, one of the one or more medical devices may be used for both imaging and treatment simultaneously. In some embodiments, the imaging and treatment processes may also be performed by multiple different medical devices. However, these changes and modifications will not depart from the scope of the present disclosure.

FIG. 2 is a schematic diagram of hardware and/or software of an exemplary computing device according to some embodiments of the present disclosure.

As shown in FIG. 2, the computing device 200 may include a processor 210, a memory 220, an input/output 230, and a communication port 240.

The processor 210 may execute computing instructions (program code) and perform the functions of the magnetic resonance image reconstruction system 100 described in the present disclosure. The computing instructions may include programs, components, data structures, procedures, modules, and functions (the functions refer to specific functions described in the present disclosure). For example, the processor 210 may process data obtained from any component of the magnetic resonance image reconstruction system 100. For example, the processor 210 may analyze the real-time motion signal of the object acquired from the motion signal acquisition device 112, and determine the data acquisition mode of each phase. As another example, the processor 210 may classify the tagged original data acquired from the imaging device 110 to obtain data of multiple categories, and perform image reconstruction on the data of multiple categories to obtain a 4D MR image of the object. In some embodiments, the processor 210 may include a microcontroller, a microprocessor, a reduced instruction set computer (RISC), an disclosure specific integrated circuit (ASIC), an disclosure specific instruction set processor (ASIP), a central processing unit (CPU), a graphics processing unit (GPU), a physical processing unit (PPU), a microcontroller unit, a digital signal processor (DSP), a field programmable gate array (FPGA), an advanced RISC machine (ARM), a programmable logic device, and any circuit and processor capable of performing one or more functions, etc., or any combination thereof. For illustration purposes only, the computing device 200 in FIG. 2 depicts only one processor, but it should be noted that the computing device 200 in the present disclosure may also include multiple processors.

The memory 220 may store data/information obtained from any other component of the magnetic resonance image reconstruction system 100. In some embodiments, the memory 220 may include mass storage, removable storage, volatile read-and-write memory, and ROM, etc., or any combination thereof.

The input/output 230 may be used to input or output signals, data, or information. In some embodiments, the input/output 230 may allow a user to interact with the magnetic resonance image reconstruction system 100. In some embodiments, the input/output 230 may include an input device and an output device. The communication port 240 may be connected to a network for data communication. The connection may be a wired connection, a wireless connection, or a combination of both. The wired connection may include a cable, an optical cable, or a telephone line, etc., or any combination thereof. The wireless connection may include one or any combination of Bluetooth^{™}, Wi-Fi, WiMax, WLAN, ZigBee^{™}, a mobile network (e.g., 3G, 4G, or 5G, etc.), etc. In some embodiments, the communication port 240 may be a standardized port, such as RS232, RS485, etc. In some embodiments, the communication port 240 may be a specially designed port. For example, the communication port 240 may be designed according to the Digital Imaging and Communications in Medicine (DICOM) protocol.

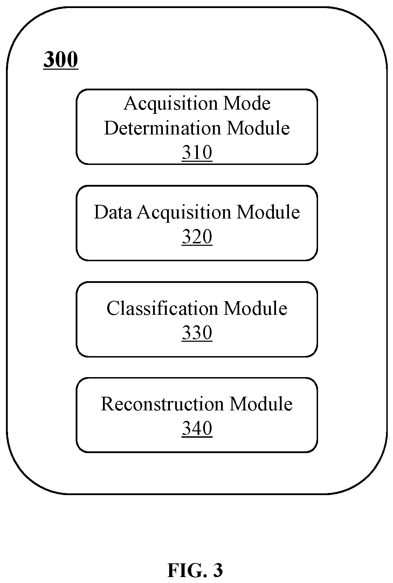

FIG. 3 is a schematic diagram of modules of an exemplary 4D MR image reconstruction apparatus according to some embodiments of the present disclosure.

As shown in FIG. 3, in some embodiments, the 4D MR image reconstruction apparatus 300 may include an acquisition method determination module 310, a data acquisition module 320, a classification module 330, and a reconstruction module 340. In some embodiments, the functions corresponding to the 4D MR image reconstruction apparatus 300 may be executed and implemented by the processing device 130 or the processor 210.

The acquisition method determination module 310 may be used to determine the data acquisition mode of each phase on the basis of the real-time motion signal of the object. In some embodiments, the data acquisition mode may include performing full sampling or edge sampling during a stable motion phase of the real-time motion signal, and performing central sampling during other phases. In some embodiments, one signal cycle of the real-time motion signal may include multiple different phases, and at least two of the multiple different phases correspond to different echo train lengths or phase encoding steps.

In some embodiments, the acquisition method determination module 310 may be used to determine the data acquisition mode of each phase on the basis of the real-time motion signal and a signal analysis model.

The data acquisition module 320 may be used to acquire original data of the object according to the data acquisition mode. In some embodiments, during the data acquisition process, the data acquisition module 320 may tag the motion phase or amplitude corresponding to each portion of data in the original data, to obtain tagged original data.

The classification module 330 may be used to classify the original data to obtain data of multiple categories, wherein data of each category corresponds to a different phase. In some embodiments, the classification module 330 may classify the original data on the basis of the tags to obtain data of multiple categories.

The reconstruction module 340 may be used to perform image reconstruction on the data of multiple categories to obtain a 4D MR image.

In some embodiments, the reconstruction module 340 may be used to perform k-space stitching on the data of multiple categories to obtain k-space data for each phase; for each phase, perform image reconstruction on the basis of the k-space data to obtain a corresponding reconstructed image; and combine the reconstructed images of each phase to obtain a 4D MR image.

In some embodiments, for each undersampled target phase, the reconstruction module 340 may supplement the unacquired data in the target phase on the basis of the sampled data from the stable motion phase, to obtain full k-space data for the target phase. In some embodiments, for each undersampled target phase, the reconstruction module 340 may supplement the unacquired data of the target phase on the basis of the sampled data of each phase between the target phase and the stable motion phase, to obtain full k-space data for the target phase.

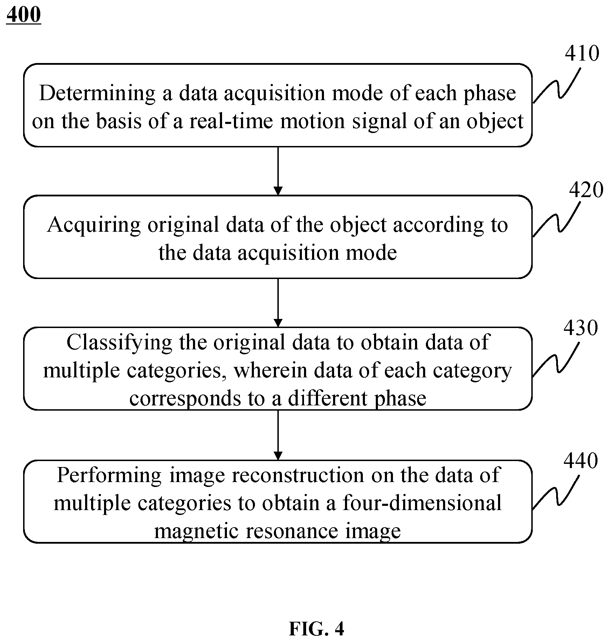

More contents regarding the acquisition method determination module 310, the data acquisition module 320, the classification module 330, and the reconstruction module 340 can be found elsewhere in the present disclosure, for example, in FIG. 4 and its related description.

It should be understood that the system and its modules shown in FIG. 3 may be implemented using various methods. For example, in some embodiments, the system and its modules may be implemented through hardware, software, or a combination of software and hardware.

It should be noted that the above description of the 4D MR image reconstruction apparatus 300 and its modules is for convenience of description and illustration only, and is not intended to limit the present disclosure to the scope of the illustrated embodiments. It is understandable that for those skilled in the art, after understanding the principles of the system, various modules may be arbitrarily combined, or subsystems may be formed to connect with other modules, without departing from these principles. Then, such variations are all within the scope of protection of this disclosure.

FIG. 4 is a schematic flowchart of an exemplary 4D MR image reconstruction method of some embodiments of this disclosure. In some embodiments, process 400 may be performed by the magnetic resonance image reconstruction system 100 (e.g., the processing device 130 in the magnetic resonance image reconstruction system 100) or the 4D MR image reconstruction apparatus 300. For example, process 400 may be stored in the form of a program or instructions in a storage device (e.g., storage device 150, a storage unit of the system). When a processor or the modules shown in FIG. 3 execute the program or instructions, process 400 may be implemented. As shown in FIG. 4, in some embodiments, process 400 may include the following steps.

At step 410, a data acquisition mode of each phase is determined on the basis of a real-time motion signal of an object. In some embodiments, step 410 may be performed by the processing device 130 or the acquisition method determination module 310.

A motion signal refers to a signal reflecting motion information such as respiration or heartbeat of an object, which can represent changes in the respiratory amplitude or heartbeat amplitude of the object over a period of time. In some embodiments, the motion signal may be presented in the form of a curve. For example, the respiratory signal may be a sine wave or cosine wave curve. In some embodiments, the trough in the respiratory signal curve may correspond to the end of the expiratory phase, and the peak may correspond to the end of the inspiratory phase. In some embodiments, the trough in the respiratory signal curve may correspond to the end of the inspiratory phase, and the peak may correspond to the end of the expiratory phase.

In some embodiments, the real-time motion signal of the object may be acquired via a motion signal acquisition device (e.g., motion signal acquisition device 112). In some embodiments, the acquisition of the real-time motion signal of the object may be performed synchronously with the scan. For example, while the imaging device 110 is scanning the object, the motion signal acquisition device 112 simultaneously acquires the real-time motion signal of the object.

A phase refers to different states during motion. For example, phases corresponding to the respiratory signal may include early inspiration, end inspiration, early expiration, end expiration; or early inspiration, mid inspiration, end inspiration, early expiration, mid expiration, and end expiration, etc.

In some embodiments, the processing device 130 may divide at least a portion of the motion signal (e.g., one signal cycle, multiple signal cycles, half a signal cycle, a part of one or more signal cycles, etc.) into multiple phases on the basis of the signal cycle of the real-time motion signal.

Taking the respiratory signal as an example, a signal cycle may refer to a signal interval between two adjacent peaks in the respiratory signal curve, a signal interval between two adjacent troughs, a signal interval that includes a pair of adjacent peak and trough, a signal interval between two non-adjacent peaks or troughs, or any other signal interval(s) that can reflect the change cycle of the respiratory signal.

It is understandable that for the same patient, the signal cycle of their motion signal is a time value whose duration is constant or substantially constant. In some embodiments, the signal cycle of the motion signal is affected by factors such as age, gender, body size, respiratory muscle strength, and lung and chest wall elasticity.

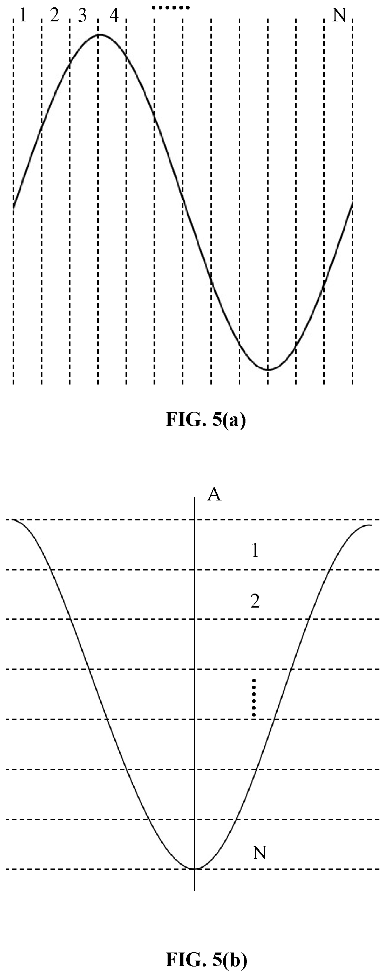

In some embodiments, the processing device 130 may divide at least a portion of the motion signal into multiple phases according to phase. For example, as shown in FIG. 5A, taking a signal interval containing a pair of adjacent peak and trough as one signal cycle of the respiratory signal, the processing device 130 may divide one signal cycle of the respiratory signal into N phases according to phase.

In some embodiments, the processing device 130 may divide at least a portion of the motion signal into multiple segments according to motion amplitude, and determine multiple phases corresponding to the multiple segments respectively. For example, as shown in FIG. 5B, taking the signal interval between two adjacent peaks as one signal cycle of the respiratory signal, the processing device 130 may, according to the respiratory amplitude, divide the portion between every two dotted lines into the same phase, obtaining N phases.

In some embodiments, multiple segments of the signal with the same amplitude change within one signal cycle may belong to the same phase. For example, as shown in FIG. 5B, taking the signal interval between two adjacent peaks as one signal cycle of the respiratory signal, the signals in two time periods within one signal cycle of the curve that are symmetrical about the midline A (i.e., the two signal segments between every two adjacent dotted lines that are symmetrical about the midline A) may be two identical phases. In some embodiments, multiple segments of the signal with the same amplitude change within one signal cycle may be different phases. For example, as shown in FIG. 5A, taking a signal interval containing a pair of adjacent peak and trough as one signal cycle of the respiratory signal, two locations within one signal cycle of the curve with the same amplitude change, i.e., two time periods symmetrical about the midline at the peak or trough, such as phase 3 and phase 4 in FIG. 5A, may be two different phases, e.g., phase 3 is end inspiration, and phase 4 is early expiration.

In some embodiments, the processing device 130 may perform equal division, regular division, or random division on at least a portion of the real-time motion signal. For example, the processing device 130 may equally divide one signal cycle of the motion signal into N segments, and the time periods corresponding to the N segments are N phases. For another example, the processing device 130 may divide one signal cycle of the motion signal into N phases in a gradually increasing manner according to a certain rule. For another example, the processing device 130 may randomly divide one signal cycle of the motion signal into N phases.

In conjunction with the above, under the synergistic effect of the main magnetic field/gradient magnetic field and the RF pulse, magnetic resonance signals related to the object can be generated according to the pulse sequence. An MRI pulse sequence is essentially the temporal arrangement of RF pulses and gradient field changes. Therefore, each pulse sequence will have time-related concepts, mainly including repetition time (TR), echo time (TE), effective echo time (effective TE), echo train length (ETL), echo spacing (ES), number of excitation (NEX), acquisition time (TA), etc.

Repetition time refers to the time interval between two consecutive executions of a pulse sequence. For example, in an SE sequence, TR refers to the time interval between the midpoints of two adjacent 90° pulses; in a gradient echo sequence, TR refers to the time interval between the midpoints of two adjacent small-angle pulses; in an inversion recovery sequence and a fast inversion recovery sequence, TR refers to the time interval between the midpoints of two adjacent 180° inversion prepulses.

Echo time refers to the time interval from the midpoint of the pulse generating the macroscopic transverse magnetization vector to the midpoint of the echo. For example, in an SE sequence, TE refers to the time interval from the midpoint of the 90° pulse to the midpoint of the spin echo; in a gradient echo sequence, TE refers to the time interval from the midpoint of the small-angle pulse to the midpoint of the gradient echo.

Effective echo time: In a fast spin echo (FSE) sequence or echo planar imaging (EPI) sequence, multiple echoes are generated after one RF pulse excitation, filling different positions in k-space, and the TE of each echo is different. In these sequences, the time interval from the midpoint of the RF pulse to the midpoint of the echo that fills the center of k-space is called the effective TE.

Echo train length refers to the number of echoes generated, acquired, and filled into one k-space after one 90° pulse excitation. The presence of an echo train can proportionally reduce the number of TR repetitions. Different phase encoding gradient fields are applied to each echo in the echo train, filling different positions in the phase encoding direction of k-space.

Echo spacing refers to the time interval between the midpoints of two adjacent echoes in an echo train. The smaller the ES, the less time is required to acquire the entire echo train.

Number of excitations, also known as number of signal averaged (NSA) or number of acquisitions (NA), refers to the number of repetitions of each phase encoding step in a pulse sequence.

Acquisition time (TA), also known as scan time, refers to the time required for the entire pulse sequence to complete signal acquisition.

In combination with the above, the data acquisition mode can reflect the aforementioned time-related parameter information of the corresponding pulse sequence during data acquisition in each phase.

In some embodiments, the data acquisition mode may include performing full sampling during the stable motion phase of a real-time motion signal, and performing central sampling during other phases.

Stable motion phase can refer to a phase where motion is stable or substantially stable. "Motion-stable" means that the amplitude change of the motion signal is 0 or less than a preset value within a certain time period, such as the difference between the motion amplitude at the start time and the motion amplitude at the end time within the time period being 0 or the absolute value being less than a first preset value (e.g., the first preset value is in the range of 0.1~1.5), or the absolute value of the difference between the maximum amplitude and the minimum amplitude within the time period being less than a second preset value (e.g., the second preset value is in the range of 1.5~3). Correspondingly, other phases refer to phases where the respiratory amplitude changes relatively significantly.

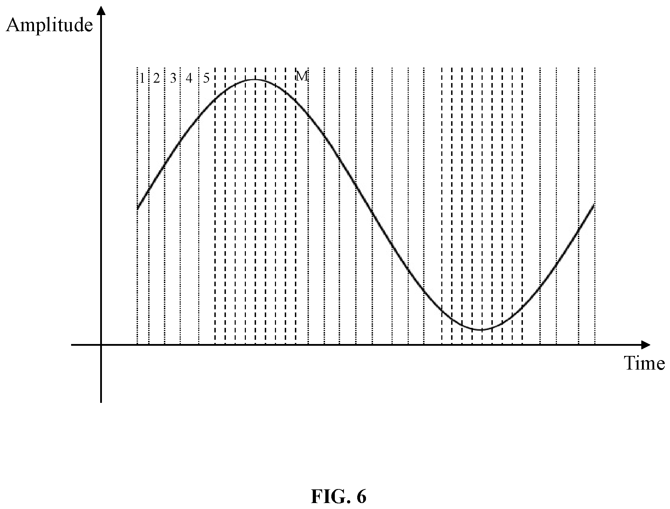

FIG. 6 is a schematic diagram of phases of an exemplary respiratory signal according to some embodiments of this disclosure, wherein the horizontal coordinate represents time, the vertical coordinate represents the amplitude of the respiratory signal, and the curve in the figure represents a portion of the respiratory signal. As shown in FIG. 6, if the portion between every two adjacent vertical dashed lines (including dotted lines and dash-dot lines) corresponds to one phase, then for the phase corresponding to the dash-dot line portion, the respiratory amplitude change is small, belonging to the stable motion phase; for the phase corresponding to the dotted line portion, the respiratory amplitude change is relatively large, belonging to other phases.

In some embodiments, one signal cycle may contain one or more stable motion phases, and each stable motion phase may contain one or more adjacent phases. For example, the processing device 130 may determine the portion of the real-time respiratory signal with small amplitude changes (such as the dash-dot line portion in FIG. 6) as one respiration-stable phase; or divide the portion with small amplitude changes into two phases on the basis of the expiration phase and inspiration phase (such as end-expiration and early inspiration, or end-inspiration and early expiration), both of which belong to the respiration-stable phase.

Full sampling means that the acquired data can completely fill k-space. Central sampling means that the acquired data can fill the central region of k-space, while no data is acquired for other regions.

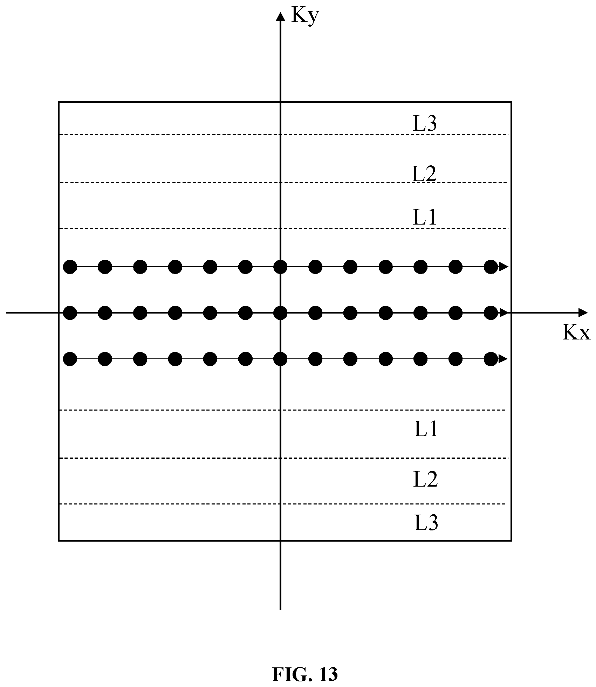

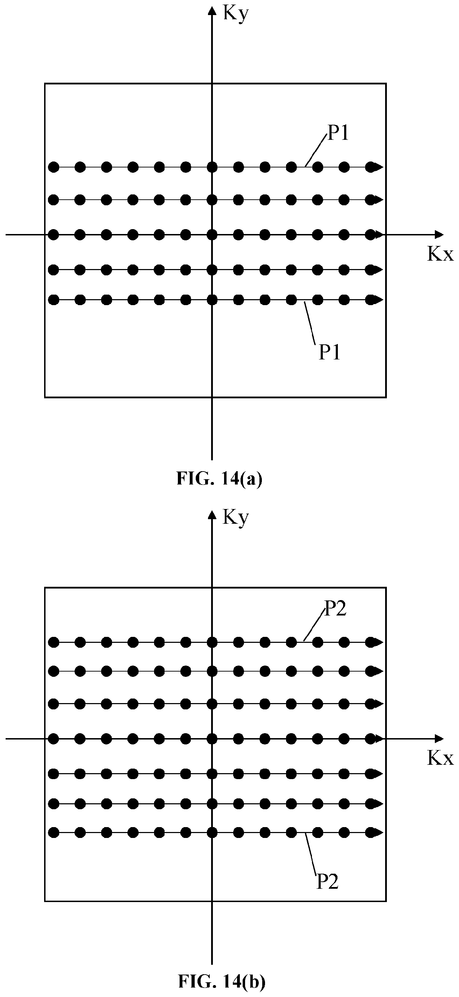

The central region of k-space refers to the area covering the centerline of k-space parallel to the horizontal coordinate but not reaching the periphery of k-space, and may include areas above and/or below the centerline. In some embodiments, after the data obtained from central sampling fills the central region of k-space, the filled region may account for 30% to 60% of the entire k-space area. In some embodiments, the proportion of the filled region in k-space occupied by central sampling data corresponding to different phases relative to the entire k-space area may be the same or different. For example, after the central sampling data corresponding to phase 1 fills the central region of k-space, the filled region as shown in FIG. 13 can be obtained; after the central sampling data corresponding to phase 2 fills the central region of k-space, the filled region as shown in FIG. 14(a) can be obtained.

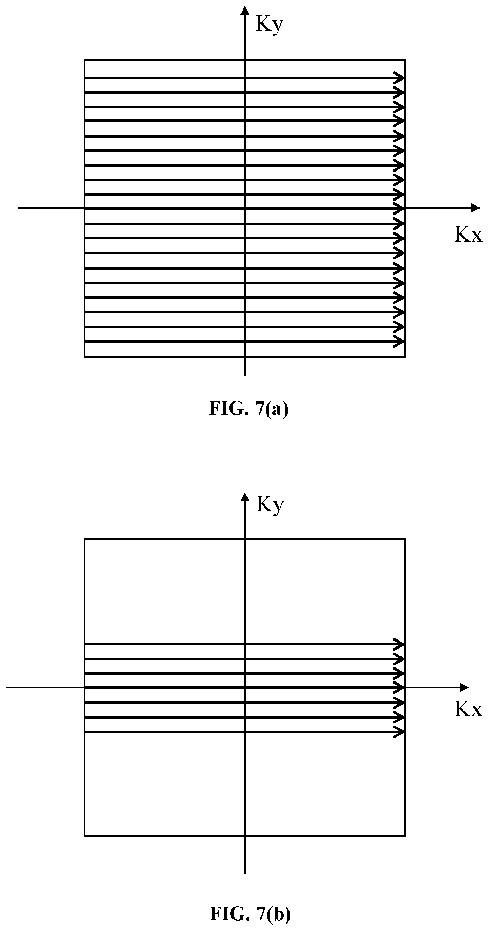

FIG. 7(a) and FIG. 7(b) are schematic diagrams of k-space filling for exemplary Cartesian acquisition according to some embodiments of this disclosure, wherein the vertical coordinate (Ky) represents the phase encoding PE direction, the horizontal coordinate represents the echo time TE, and the lines with arrows represent acquired data. FIG. 7(a) shows k-space corresponding to full sampling, and FIG. 7(b) shows k-space corresponding to central sampling.

It should be noted that FIG. 7(a) and FIG. 7(b) are only examples. In some embodiments, the number of acquisition lines for central sampling can be adjusted according to actual needs, such as 2, 3, 4 lines including the center, etc. This disclosure does not impose specific limitations on this.

In some embodiments, the data acquisition mode may include performing peripheral sampling during the stable motion phase of a real-time motion signal, and performing central sampling during other phases. Peripheral sampling means that the acquired data fills the peripheral part of k-space, which corresponds to regions other than the central region of k-space (such as the blank areas in FIG. 7(b)). In some embodiments, after the data obtained from peripheral sampling fills k-space, from the periphery of k-space towards the centerline parallel to the horizontal coordinate, it may account for 20% to 35% of the entire k-space area. In some embodiments, the data obtained from peripheral sampling can fill the upper and lower edges of k-space respectively. In some embodiments, the data filling the upper and lower edges of k-space may account for 40% to 70% of the entire k-space area. For example, after the data obtained from peripheral sampling fills k-space, the data at the upper edge of k-space accounts for 20% of the entire k-space area, and the data at the lower edge of k-space accounts for 20% of the entire k-space area.

In some embodiments, the real-time motion signal may include multiple different phases, and the echo train lengths corresponding to at least two of the multiple different phases are different. For example, early expiration and end-expiration may correspond to different echo train lengths respectively.

In some embodiments, the echo train length of a phase is related to the length of the initial echo train. In some embodiments, the echo train lengths corresponding to the at least two phases may respectively account for different proportions of the length of the initial echo train. For example, if the initial echo train in the determined pulse sequence contains 7 echo signals, the echo train corresponding to early expiration may be the first 3 echo signals in the initial echo train, and the echo train corresponding to end-expiration may be the first 5 or all echo signals in the initial echo train.

In some embodiments, the echo train length of the stable motion phase may be greater than the echo train length of other phases. For example, the echo train corresponding to mid-expiration contains 3 echo signals, and the echo train corresponding to end-expiration contains 5 echo signals. Generally, near the peak or trough of the signal curve, the amplitude change of the respiratory signal is small, i.e., the respiration is stable. The peak or trough of the signal curve may correspond to end-expiration or end-inspiration.

In some embodiments, when the echo train length of the stable motion phase is greater than the echo train length of other phases, the echo train length of each of the other phases may be the same. For example, the echo train of the respiration-stable phase contains 4 echo signals, and the echo trains of other phases all contain 2 echo signals.

In some embodiments, the echo train length of each of the multiple different phases may all be different, and the echo train length of the stable motion phase is the largest. In some embodiments, the echo train lengths corresponding to multiple phases before or after the stable motion phase may gradually increase or decrease according to the temporal order of the motion signal, and the echo train length of the stable motion phase is the largest. Exemplarily, with reference to FIG. 6, the echo train lengths corresponding to the 5 phases before the dash-dot line may gradually increase, i.e., echo train length of phase 5 > echo train length of phase 4 > echo train length of phase 3 > echo train length of phase 2 > echo train length of phase 1, and the echo train length of the long dash-dot-dot line portion is greater than the echo train length of phase 5. In some embodiments, the echo train lengths corresponding to multiple phases before or after the stable motion phase may be randomly distributed. For example, referring to FIG. 6, the echo train length for phase 1 is 2, for phase 2 is 5, for phase 3 is 4, and for phase 4 is 3, etc.

As described above, one signal cycle of the real-time motion signal may contain two or more identical phases, such as two or more signal portions with the same amplitude variation. In some embodiments, when a signal cycle of the real-time motion signal contains identical phases, the echo train lengths corresponding to two identical phases located in different time periods may be the same or different. For example, as shown in FIG. 6, assuming that phase 5 and phase M on either side of the dashed line are identical phases, such as both being mid-respiratory phases, the echo trains corresponding to phase 5 and phase M may both contain 3 echo signals; or the echo train for phase 5 contains 4 echo signals, and the echo train for phase M contains 3 echo signals.

In some embodiments, among multiple different phases within one signal cycle of the real-time motion signal, at least two phases correspond to different phase encoding steps. Phase encoding steps refer to the number of phase encoding gradients with different current intensities and durations that are executed to complete k-space data filling. In some embodiments, the phase encoding step for the stable motion phase can be larger than that for other phases, and the phase encoding steps for the other phases can all be the same. In some embodiments, the phase encoding step for each of the multiple different phases can be different, and the phase encoding step for the stable motion phase is the largest.

For example, as shown in FIG. 6, if the vertical dashed lines (including dotted lines and dashed lines) in the figure represent phase encoding steps, within the same time length, the distribution of dashed lines is denser, while the distribution of dotted lines is sparser. That is, the dashed line portion corresponds to the phase encoding step when the respiratory amplitude variation is low (i.e., stable breathing), meaning the phase encoding step is larger in this stage, allowing more data to be acquired from the edge positions of k-space, thereby obtaining more image details and increasing image information redundancy; the dotted line portion corresponds to the phase encoding step when the respiratory amplitude variation is large, meaning the phase encoding step is smaller in this stage, allowing faster acquisition of data from the central region of k-space, thereby obtaining higher image contrast.

The determination of the phase encoding step corresponding to each phase can be similar to the echo train length. More details can be seen above.

In some embodiments, the processing device 130 can determine the data acquisition mode of each phase on the basis of the real-time motion signal and a signal analysis model. The input of the signal analysis model is the real-time motion signal, and the output is the data acquisition mode. For example, the processing device 130 can input the acquired real-time motion signal of the object into a trained signal analysis model. After analysis and processing, the signal analysis model outputs the number of phases contained in the real-time motion signal, the time information and/or amplitude information for each phase, and the echo train length (and/or phase encoding step) corresponding to each phase.

In some embodiments, the signal analysis model can be obtained by training on the basis of pre-acquired sample motion signals. The training labels include sample phases of the sample motion signals and/or sample echo train lengths (or sample phase encoding steps) corresponding to each sample phase. For example, the sample motion signals can be used as training input to an initial model, and the sample phases of the sample motion signals and the sample echo train lengths corresponding to each sample phase are used as training labels. The trained signal analysis model is obtained through iterative training of the initial model. In some embodiments, during the training process, the model can be optimized by constructing a loss function. For example, a loss function is constructed on the basis of the prediction results of the initial model and the training labels. When the loss function value converges to a target value, the training ends, and the trained signal analysis model is obtained.

In some embodiments, the signal analysis model may include a neural network. For example, the neural network may include a Convolutional Neural Network (CNN), U-Net, Recurrent Neural Network (RNN), Graph Neural Networks (GNN), etc.

At step 420, original data of the object are acquired according to the data acquisition mode. In some embodiments, step 420 can be performed by the processing device 130 or the data acquisition module 320.

In some embodiments, the processing device 130 can perform data acquisition in each phase of the real-time motion signal according to the determined echo train length (or phase encoding step) to obtain original data of the object.

In some embodiments, during the data acquisition process, the processing device 130 can synchronously mark the motion phase or amplitude corresponding to each portion of data in the original data to obtain markings for the original data. For example, when the processing device 130 performs data acquisition in each respiratory phase according to the determined echo train length (or phase encoding step), it can synchronously mark the respiratory signal amplitude or phase corresponding to the currently acquired data.

In some embodiments, the processing device 130 can mark the acquired original data after completing the acquisition of all original data of the object. In some embodiments, the processing device 130 can mark the acquired portion after completing the acquisition of a portion of the original data of the object, and then proceed with the acquisition of other portions of data.

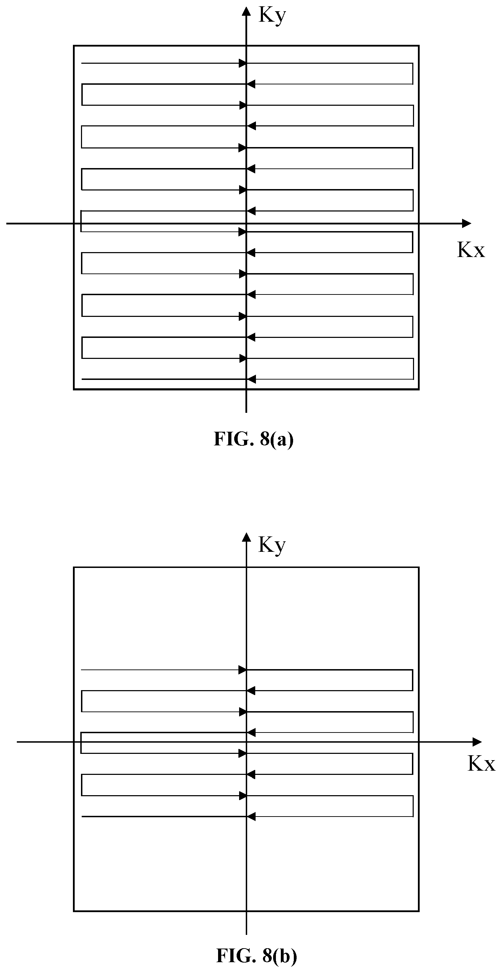

In some embodiments, the processing device 130 can use a fast sequence to acquire original data of the object. For example, fast sequences may include GRE sequences, EPI sequences, etc. Data acquisition on the basis of the EPI sequence can also be referred to as the Zig-Zag acquisition mode. FIGS. 8(a) and 8(b) are schematic diagrams of exemplary Zig-Zag acquisition k-space filling according to some embodiments of this disclosure. For example, as shown in FIGS. 8(a) and 8(b), the lines with arrows forming a zigzag pattern represent acquisition lines, and the rectangular box represents the full k-space. The acquisition lines shown in FIG. 8(a) can correspond to the data to be acquired during the stable motion phase, and the acquisition lines shown in FIG. 8(b) can correspond to the amount of data to be acquired for other phases (e.g., phases with large motion amplitude variation). In some embodiments, the processing device 130 can use other sequences to acquire original data of the object, and this disclosure is not specifically limited in this regard.

In some embodiments, the data acquisition mode for the original data of the object may include at least one of the following: Cartesian acquisition, Zig-Zag acquisition, radial acquisition, spiral acquisition, or windmill acquisition. The data acquisition modes described here are for different objects; for different objects, the same or different data acquisition modes can be used, but the same acquisition method is typically used throughout the entire data acquisition process for the same object. For example, the Cartesian acquisition method can be used for the same object to sample original data for each phase according to the determined echo train length and/or phase encoding step. In some embodiments, different acquisition methods such as Cartesian, radial, spiral, or windmill acquisition can be achieved by changing the encoding.

It is understandable that the data filling method used during k-space filling corresponds to the acquisition method used during original data sampling. For example, when Cartesian sampling is used for central or peripheral data for each phase, correspondingly, Cartesian filling will be used to fill the k-space with the original data of each phase.

FIGS. 7(a) and 7(b) are schematic diagrams of exemplary Cartesian acquisition k-space filling according to some embodiments of this disclosure. The lines with arrows represent acquired data, and the rectangular box represents the full k-space. The acquisition lines shown in FIG. 7(a) correspond to the data to be acquired during the stable motion state, and the acquisition lines shown in FIG. 7(b) correspond to the amount of data to be acquired when the motion amplitude varies significantly.

FIGS. 8(a) and 8(b) are schematic diagrams of K-space filling for an exemplary Zig-Zag acquisition according to some embodiments of the present disclosure. The lines with arrows forming a rectangular spiral represent acquisition lines, and the rectangular box represents the full K-space. The acquisition lines shown in FIG. 8(a) may correspond to data to be acquired during a stable motion state, while the acquisition lines shown in FIG. 8(b) may correspond to data to be acquired when the motion amplitude varies significantly.

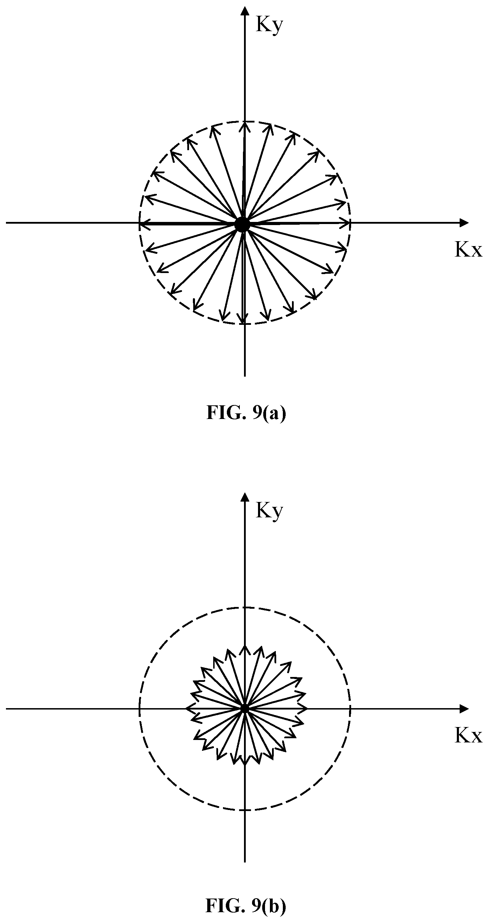

FIGS. 9(a) and 9(b) are schematic diagrams of K-space filling for an exemplary radial acquisition according to some embodiments of the present disclosure. The vertical coordinate (Ky) represents the phase encoding (PE) direction, and the horizontal coordinate represents the echo time (TE). The radial lines with arrows represent the acquired data, and the dashed circular frame represents the full K-space. The acquisition lines shown in FIG. 9(a) correspond to data to be acquired during a stable motion state, while the acquisition lines shown in FIG. 9(b) correspond to the amount of data to be acquired when the motion amplitude varies significantly.

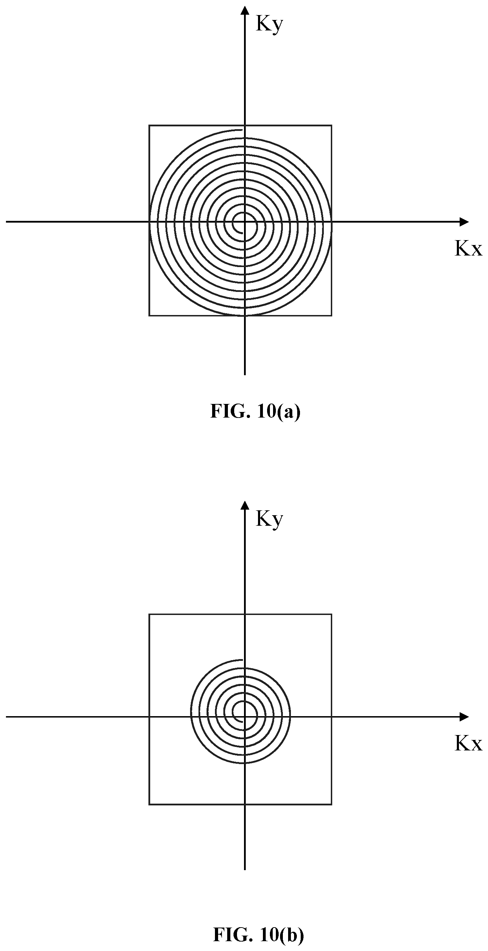

FIGS. 10(a) and 10(b) are schematic diagrams of K-space filling for an exemplary spiral acquisition according to some embodiments of the present disclosure. The vertical coordinate (Ky) represents the phase encoding (PE) direction, and the horizontal coordinate represents the echo time (TE). The spiral lines represent the acquired data, and the rectangular box represents the full K-space. The acquisition lines shown in FIG. 10(a) correspond to data to be acquired during a stable motion state, while the acquisition lines shown in FIG. 10(b) correspond to the amount of data to be acquired when the motion amplitude varies significantly.

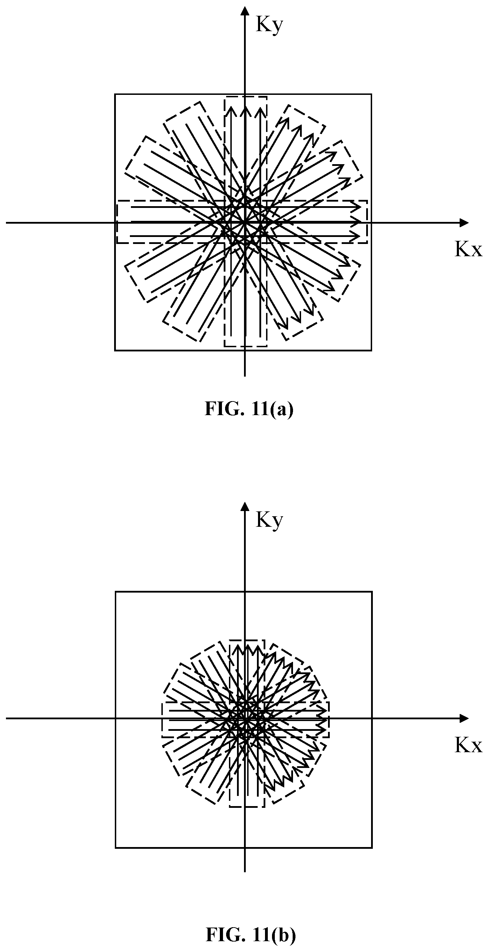

FIGS. 11(a) and 11(b) are schematic diagrams of K-space filling for an exemplary windmill acquisition according to some embodiments of the present disclosure. The vertical coordinate (Ky) represents the phase encoding (PE) direction, and the horizontal coordinate represents the echo time (TE). The solid rectangular box represents the full K-space, and the lines with arrows represent acquisition lines. Every 3 acquisition lines form one blade of the windmill (as shown in the dashed box), each blade corresponds to one acquisition, and multiple blades form a star-like shape. The acquisition lines shown in FIG. 11(a) correspond to data to be acquired during a stable motion state, while the acquisition lines shown in FIG. 11(b) correspond to the amount of data to be acquired when the motion amplitude varies significantly.

In some embodiments, the data acquisition modes for the original data of the object include, but are not limited to, 2D acquisition and 3D acquisition. For example, 2D Cartesian acquisition, 3D Cartesian acquisition, 2D radial acquisition, 3D radial acquisition, 2D spiral acquisition, 3D spiral acquisition, 2D windmill acquisition, 3D windmill acquisition, etc.

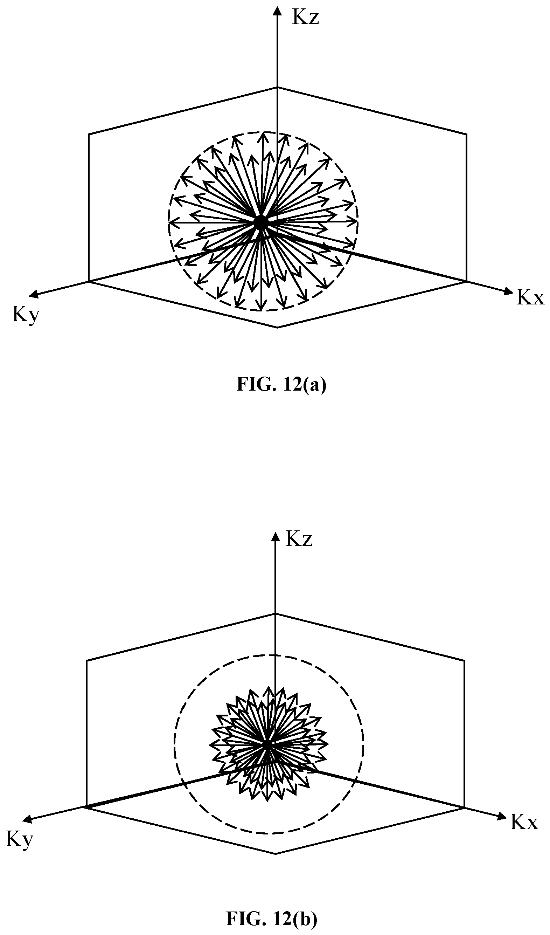

FIGS. 12(a) and 12(b) are schematic diagrams of K-space filling for an exemplary radial 3D acquisition according to some embodiments of the present disclosure. The corresponding K-space is also 3-dimensional. The radial lines with arrows represent the acquired data, and the dashed circular frame represents the full K-space. The acquisition lines shown in FIG. 12(a) correspond to data to be acquired during a stable motion state, while the acquisition lines shown in FIG. 12(b) correspond to the amount of data to be acquired when the motion amplitude varies significantly.

At step 430, the original data is classified to obtain data of multiple categories. In some embodiments, step 430 may be performed by the processing device 130 or the classification module 330.

Among the multiple types obtained by classification, data of each category corresponds to a different phase. In some embodiments, the processing device 130 may classify data with the same motion amplitude or phase into one type according to the marked motion amplitude or phase, thereby obtaining data of multiple categories.

In some embodiments, the processing device 130 may classify the original data according to the data acquisition mode. For example, when the echo train length corresponding to each phase is different, the processing device 130 may classify the original data acquired using the same echo train length into one category.

In some embodiments, the processing device 130 may perform data classification synchronously during the data acquisition process. For example, during the process of acquiring the original data of the object according to the determined data acquisition mode, the processing device 130 may bin and store the data acquired in different phases, thereby obtaining multiple types of original data.

At step 440, image reconstruction is performed on the data of multiple categories to obtain the four-dimensional magnetic resonance image. In some embodiments, step 440 may be performed by the processing device 130 or the reconstruction module 340.

In some embodiments, the processing device 130 may perform K-space stitching on the data of multiple categories to obtain K-space data for each phase. Further, for each phase, the processing device 130 may perform image reconstruction on the basis of the K-space data of that phase to obtain a corresponding reconstructed image; and combine the reconstructed images of each phase to obtain a 4D MR image. In some embodiments, K-space may include, but is not limited to, 2D K-space (as shown in FIGS. 7(a)-11(b)) and 3D K-space (as shown in FIGS. 12(a) and 12(b)).

K-space stitching refers to filling the acquired data into K-space, such as obtaining the K-space data shown in FIGS. 7(a)-12(b), or parts of the K-space data shown in FIGS. 7(a)-12(b).

In some embodiments, for each of the obtained multiple types, the processing device 130 may fill the data corresponding to that category into K-space, thereby obtaining the K-space data for the phase corresponding to that category. In some embodiments, the processing device 130 may obtain the full K-space data for each phase by sharing data from the original data corresponding to each type.

In some embodiments, for each undersampled target phase, the processing device 130 may supplement the unacquired data in the target phase on the basis of the sampled data from the stable motion phase, to obtain the full K-space data for the target phase.

An undersampled target phase is a phase where the acquired data is insufficient to completely fill the K-space. For example, for a phase with significant motion amplitude variation, due to the use of a shorter echo train length or phase encoding step for data acquisition, the amount of sampled data obtained is small and cannot completely fill the K-space, e.g., only enough to fill the central region of the K-space.

In some embodiments, when full sampling is performed during the stable motion phase, the processing device 130 may supplement the unacquired data in the target phase on the basis of the sampled data from the stable motion phase, to obtain the full K-space data for the target phase. In some embodiments, the processing device 130 may determine the target data not acquired in the target phase, obtain the target data from the acquired data of the stable motion phase, and perform K-space filling on the basis of the sampled data of the target phase and the target data, thereby obtaining the full K-space data for the target phase.

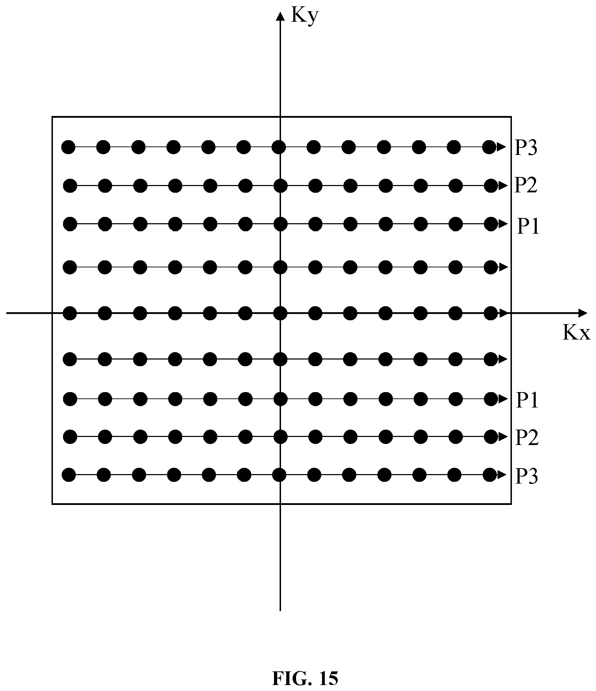

FIGS. 13-14(b) are schematic diagrams of exemplary undersampled K-space filling according to some embodiments of the present disclosure. FIG. 15 is a schematic diagram of exemplary fully sampled K-space filling according to some embodiments of the present disclosure. The solid rectangular box in the figures represents the size of the full K-space. Exemplarily, taking the 2D Cartesian acquisition method as an example, with reference to FIGS. 13 and 15, when full sampling is performed during the stable motion phase, after K-space filling on the basis of the acquired original data (also referred to as sampled data), full K-space data similar to that shown in FIG. 15 can be obtained. However, for a phase with significant motion amplitude variation (i.e., the target phase), due to undersampling, if the acquired original data is directly used for K-space filling, K-space data similar to that shown in FIG. 13, 14(a), or 14(b) is obtained. At this point, the processing device 130 may obtain the data not acquired in the target phase from the sampled data of the stable motion phase, and fill the obtained data and the sampled data of the target phase into K-space, thereby obtaining the full K-space data for the target phase (e.g., K-space data similar to that shown in FIG. 15). For example, taking the undersampled target phase shown in FIG. 13 as an example, the processing device 130 may obtain the data corresponding to rows P1, P2, and P3 in FIG. 15, and fill the data of rows P1, P2, and P3 into the positions of dashed lines L1, L2, and L3 shown in FIG. 13, respectively, to obtain the full K-space data for the target phase.

In some embodiments, when edge sampling is performed during the stable motion phase, the processing device 130 may supplement the unacquired data in the target phase on the basis of the edge sampling data from the stable motion phase, to obtain the full K-space data for the target phase. Accordingly, the processing device 130 can supplement the unacquired data (e.g., data in the central region of k-space) during the stable motion phase on the basis of sampled data from other phases, to obtain full k-space data for the stable motion phase. In some embodiments, the processing device 130 can supplement the unacquired data during the stable motion phase on the basis of sampled data from one or more other phases adjacent to the stable motion phase.

Data in the central region of k-space determines the contrast of the reconstructed image, while data at the edges of k-space determines the details of the reconstructed image. The stable motion phase, where full sampling or edge sampling is performed, acquires more high-dimensional data (i.e., image detail information). The target phase where central sampling is performed (i.e., the phase with larger motion amplitude variations) acquires central region data (i.e., image contrast information). By sharing the original data acquired during the stable motion phase with the target phase, scanning efficiency can be improved while enabling the target phase to possess both image detail information and central contrast information, thereby resulting in higher image quality for the reconstructed image of each phase.

In some embodiments, for each undersampled target phase, the processing device 130 can supplement the unacquired data of the target phase on the basis of the stable motion phase and one or more candidate phases with more sampled data than the target phase, to obtain full k-space data for the target phase. For example, the processing device 130 can first supplement the target phase with the sampled data, which exceeds that of the target phase, from phases that have more sampled data than the target phase, and then obtain other unacquired data for the target phase from the stable motion phase to supplement the target phase, thereby obtaining complete data for the target phase.

In some embodiments, for each undersampled target phase, the processing device 130 can supplement the unacquired data of the target phase on the basis of the sampled data of each phase between the target phase and the stable motion phase, to obtain full k-space data for the target phase.

In some embodiments, when the echo train lengths (or phase encoding steps) corresponding to multiple phases before or after the stable motion phase gradually increase or decrease over time, the processing device 130 can supplement the unacquired data of the target phase on the basis of the sampled data of each phase between the target phase and the stable motion phase.

Specifically, the processing device 130 can supplement the data of the target phase in a first part of k-space on the basis of the sampled data of a first phase adjacent to the target phase; supplement the data of the target phase in a second part of k-space on the basis of the sampled data of a second phase adjacent to the first phase; and repeat the above steps until the data of the target phase in k-space is fully supplemented on the basis of the sampled data of the stable motion phase.

Exemplarily, taking the 2D Cartesian acquisition method as an example, with reference to FIGS. 13, 14(a)-14(b), and 15, assuming that the small black dots shown in FIG. 13 represent the sampled data corresponding to the current target phase, the small black dots shown in FIG. 14(a) represent the sampled data corresponding to the first phase adjacent to the target phase, the small black dots shown in FIG. 14(b) represent the sampled data corresponding to the second phase adjacent to the first phase, wherein the first phase and the second phase are phases between the target phase and the respiratory-stable phase, and the small black dots shown in FIG. 15 represent the sampled data corresponding to the stable motion phase, which is adjacent to the second phase. The small black dots shown in the figures represent sampled data. For the target phase, the processing device 130 can fill the data corresponding to row P1 in FIG. 14(a) into the row indicated by the dotted line L1 in FIG. 13, fill the data corresponding to row P2 in FIG. 14(b) into the row indicated by the dotted line L2 in FIG. 13, and fill the data corresponding to row P3 in FIG. 15 into the row indicated by the dotted line L3 in FIG. 13, thereby obtaining the full k-space data for the target phase.

It can be understood that the above description and the undersampled k-space data in FIGS. 13-14(b) are merely examples. In some embodiments, the amount of undersampled k-space data can be greater or less than that shown in the figures, such as including 2 rows as in FIG. 13, or having one more row of data than shown in FIG. 13, etc. In some embodiments, the target phase can be the phase corresponding to the k-space data shown in FIG. 14(a) or FIG. 14(b), and this disclosure is not limited in this regard.

Since the echo train lengths (or phase encoding steps) corresponding to multiple phases before or after the stable motion phase gradually increase (or decrease) over time, the sampled data of each phase between the target phase and the stable motion phase gradually increases (or decreases). Supplementing the unacquired data of the target phase stepwise from the first phase adjacent to the target phase to the stable motion phase can ensure the authenticity of the k-space data of the target phase.

In some embodiments, when the target phase has two adjacent first phases (e.g., the adjacent phases on the left and right sides of the target phase), and the sampled data of both adjacent first phases is more than that of the target phase, the processing device 130 can supplement the data of the target phase in a first part of k-space on the basis of the sampled data of the two adjacent first phases. For example, with reference to FIGS. 13 and 14(a), the processing device 130 can average the data of row P1 of the first phase adjacent to the left side of the target phase with the data of row P1 of the first phase adjacent to the right side of the target phase, and fill the averaged data into the row indicated by the dotted line L1 in FIG. 13.

After the full k-space data is obtained for each phase on the basis of the above method, further, for each phase, the processing device 130 can perform image reconstruction on the basis of the full k-space data of that phase to obtain a corresponding reconstructed image. In some embodiments, the image reconstruction algorithm may include, but is not limited to, Fourier transform, back projection reconstruction, iterative reconstruction, and fitting approximation methods, and this disclosure is not specifically limited in this regard.

By combining the reconstructed images of each phase, a 4D MR image with temporal information can be obtained. This 4D MR image can be used for lesion diagnosis, scientific research, radiotherapy planning, etc., and this disclosure is not limited in this regard.

It should be noted that the above description of process 400 is for illustration and explanation only, and does not limit the scope of disclosure of this disclosure. For those skilled in the art, various modifications and changes can be made to process 400 under the guidance of this disclosure. However, these modifications and changes are still within the scope of this disclosure.

Some embodiments of this disclosure further provide a 4D MR image reconstruction method, comprising: determining full k-space data for each phase on the basis of acquired original data; performing image reconstruction on the basis of the full k-space data for each phase to obtain a corresponding reconstructed image; and combining the reconstructed images of each phase to obtain a 4D MR image. For each undersampled target phase, unacquired data in the target phase is supplemented on the basis of sampled data from a stable motion phase and/or sampled data from one or more candidate phases with more sampled data than the target phase, to obtain the full k-space data for the target phase.

Some embodiments of this disclosure also provide a 4D MR image reconstruction system, the system comprising at least one non-transitory storage medium including a set of instructions; and one or more processors in communication with the at least one non-transitory storage medium. When the instructions are executed, the one or more processors are configured to perform the 4D MR image reconstruction method as described above (e.g., process 400).

Some embodiments of this disclosure also provide a computer-readable non-transitory storage medium, the non-transitory storage medium storing computer instructions, and when the computer instructions are read by a computer in the non-transitory storage medium, the computer executes the 4D MR image reconstruction method as described above (e.g., process 400).

Potential beneficial effects of the embodiments of this disclosure include, but are not limited to: (1) performing full sampling or edge sampling in a state with small motion amplitude variations can reduce the impact of motion artifacts while acquiring high-dimensional data reflecting image details; (2) performing central sampling in a state with large motion amplitude variations can improve scanning efficiency while ensuring image contrast information; (3) sharing sampled data from different phases can enable each phase to possess data with high contrast and image details, thereby improving the quality of the reconstructed image for each phase; (4) tagging during the data acquisition process and classifying the acquired original data into multiple types on the basis of the tags, so that data of each category carries phase information, thus reducing the impact of motion artifacts.

The basic concepts have been described above. Obviously, for those skilled in the art, the above detailed disclosure is merely an example and does not constitute a limitation of this disclosure. Although not explicitly stated herein, those skilled in the art may make various modifications, improvements, and corrections to this disclosure. Such modifications, improvements, and corrections are suggested in this disclosure, and thus such modifications, improvements, and corrections still fall within the spirit and scope of the exemplary embodiments of this disclosure.

Meanwhile, this disclosure uses specific terms to describe the embodiments of this disclosure. For example, "one embodiment," "an embodiment," and/or "some embodiments" refer to a particular feature, structure, or characteristic associated with at least one embodiment of this disclosure. Therefore, it should be emphasized and noted that references to "an embodiment" or "one embodiment" or "an alternative embodiment" in different places of this disclosure do not necessarily refer to the same embodiment. Furthermore, certain features, structures, or characteristics of one or more embodiments of this disclosure may be combined appropriately.

Furthermore, unless explicitly stated in the claims, the order of processing elements and sequences, the use of numbers or letters, or other names in this disclosure are not intended to limit the order of the processes and methods of this disclosure. Although the above disclosure discusses some currently considered useful embodiments of the invention through various examples, it should be understood that such details are for illustrative purposes only, and the appended claims are not limited to the disclosed embodiments; on the contrary, the claims are intended to cover all modifications and equivalent combinations that fall within the spirit and scope of the embodiments of this disclosure. For example, although the system components described above may be implemented by hardware devices, they may also be implemented solely by software solutions, such as installing the described system on an existing server or mobile device.

Similarly, it should be noted that, in order to simplify the description of this disclosure to help understand one or more inventive embodiments, the foregoing description of the embodiments of this disclosure sometimes combines various features into a single embodiment, figure, or description thereof. However, this method of disclosure does not imply that the object matter of this disclosure requires more features than those recited in the claims. In fact, the features of an embodiment are fewer than all the features of a single disclosed embodiment described above.

In some embodiments, numbers describing component quantities or attributes are used, and it should be understood that such numbers used in describing the embodiments are, in some instances, modified by the terms "about", "approximately" or "substantially." Unless otherwise indicated, "about", "approximately" or "substantially" means that the stated number allows for a variation of ±20%. Accordingly, in some embodiments, the numerical parameters set forth in the disclosure and claims are approximations that may vary depending on the desired characteristics of the individual embodiments. In some embodiments, numerical parameters should consider the specified significant digits and adopt a general rounding method. Although the numerical ranges and parameters used to confirm the breadth of scope in some embodiments of this disclosure are approximations, in specific embodiments, such numerical values are set as precisely as feasible within the practical range.

Each patent, patent application, patent application publication, and other material, such as articles, books, specification, publications, documents, etc., cited in this disclosure is hereby incorporated by reference in its entirety, except for disclosure history files that are inconsistent with or conflict with the content of this disclosure, and except for documents that limit the broadest scope of the claims of this disclosure (whether currently or subsequently appended to this disclosure). It should be noted that if there is any inconsistency or conflict between the descriptions, definitions, and/or usage of terms in the supplementary materials of this disclosure and the content described in this disclosure, the descriptions, definitions, and/or usage of terms in this disclosure shall prevail.

Finally, it should be understood that the embodiments described in this disclosure are merely illustrative of the principles of the embodiments of this disclosure. Other variations may also fall within the scope of this disclosure. Therefore, by way of example and not limitation, alternative configurations of the embodiments of this disclosure may be considered consistent with the teachings of this disclosure. Accordingly, the embodiments of this disclosure are not limited to the embodiments explicitly introduced and described in this disclosure.

## Claims

1. A method for reconstructing a four-dimensional magnetic resonance image, comprising:
determining a data acquisition mode of each phase on the basis of a real-time motion signal of an object;
acquiring original data of the object according to the data acquisition mode;
classifying the original data to obtain data of multiple categories, wherein data of each category corresponds to a different phase; and
performing image reconstruction on the data of multiple categories to obtain a four-dimensional magnetic resonance image.

2. The method of claim 1, wherein the data acquisition mode comprises performing full sampling during a stable motion phase of the real-time motion signal, and performing central sampling during other phases.

3. The method of claim 1, wherein the data acquisition mode comprises performing edge sampling during a stable motion phase of the real-time motion signal, and performing central sampling during other phases.

4. The method of claim 2 or claim 3, wherein one signal cycle of the real-time motion signal includes multiple different phases, and at least two of the multiple different phases correspond to different echo train lengths.

5. The method of claim 2 or claim 3, wherein one signal cycle of the real-time motion signal includes multiple different phases, and at least two of the multiple different phases correspond to different phase encoding steps.

6. The method of claim 4 or claim 5, wherein an echo train length of the stable motion phase is greater than echo train lengths of the other phases; or
a phase encoding step of the stable motion phase is greater than phase encoding steps of the other phases.

7. The method of claim 4 or claim 5, wherein an echo train length of each of the multiple different phases is different from each other, and an echo train length of the stable motion phase is the largest; or
a phase encoding step of each of the multiple different phases is different from each other, and a phase encoding step of the stable motion phase is the largest.

8. The method of any one of claims 4-7, wherein echo train lengths or phase encoding steps of two same phases in different time periods within the one signal cycle are different.

9. The method of any one of claims 2-8, wherein the determining the data acquisition mode of each phase on the basis of the real-time motion signal of the object comprises:
determining the data acquisition mode of each phase on the basis of the real-time motion signal and a signal analysis model, the signal analysis model comprising a trained machine learning model.

10. The method of claim 9, wherein the signal analysis model is obtained by training on the basis of pre-acquired sample motion signals, and a training label of a sample motion signal of the pre-acquired sample motion signals includes sample phases of the sample motion signal and/or sample echo train lengths or sample phase encoding steps corresponding to each of the sample phases.

11. The method of any one of claims 2-10, wherein the stable motion phase includes a phase where a motion of the object is stable or substantially stable, and the stable or substantially stable motion means that an amplitude variation of motion signals within a certain time period is less than a preset value.

12. The method of any one of claims 1-11, wherein the data acquisition mode comprises at least one of: Cartesian acquisition, Zig-Zag acquisition, radial acquisition, spiral acquisition, or windmill acquisition.

13. The method of any one of claims 1-12, wherein the acquiring the original data of the object comprises:
during data acquisition, marking a motion phase or a motion amplitude corresponding to each portion of data in the original data to obtain a mark of the original data.

14. The method of any one of claims 1-13, wherein the performing image reconstruction on the data of multiple categories to obtain the four-dimensional magnetic resonance image comprises:
performing k-space stitching on the data of multiple categories to obtain k-space data for the each phase;
for the each phase, performing the image reconstruction on the basis of the k-space data to obtain a reconstructed image of the each phase; and
combining the reconstructed image of the each phase to obtain the four-dimensional magnetic resonance image.

15. The method of claim 14, wherein the performing k-space stitching on the data of multiple categories further comprises:
for each undersampled target phase,
supplementing unacquired data in the undersampled target phase on the basis of sampled data of a stable motion phase to obtain full k-space data of the undersampled target phase.

16. The method of claim 14, wherein the performing k-space stitching on the data of multiple categories further comprises:
for each undersampled target phase,
respectively supplementing unacquired data of the each undersampled target phase on the basis of sampled data of each phase between the undersampled target phase and a stable motion phase to obtain full k-space data of the target undersampled phase.

17. The method of claim 16, wherein the respectively supplementing the unacquired data of the undersampled target phase on the basis of the sampled data of each phase between the target phase and the stable motion phase comprises:
supplementing data of a first part of the undersampled target phase in k-space on the basis of sampled data of a first phase adjacent to the target phase;
supplement data of a second part of the undersampled target phase in the k-space on the basis of sampled data of a second phase adjacent to the first phase; and
repeating the above steps until data of the undersampled target phase in the k-space is fully supplemented on the basis of the sampled data of the stable motion phase.

18. A method for reconstructing a four-dimensional magnetic resonance image, comprises:
determining full k-space data for each phase on the basis of acquired original data;
for the each phase, performing image reconstruction on the basis of the full k-space data to obtain a reconstructed image of the each phase; and
combining the reconstructed image of the each phase to obtain a four-dimensional magnetic resonance image;
wherein determining the full k-space data for the each phase comprises: for each undersampled target phase, supplementing unacquired data in the undersampled target phase on the basis of at least one of sampled data of a stable motion phase or sampled data of a candidate phase having more sampled data than the undersampled target phase, to obtain the full k-space data of the undersampled target phase.

19. A system for reconstructing a four-dimensional magnetic resonance image, comprising:
at least one non-transitory storage medium comprising a set of instructions; and
one or more processors in communication with the at least one non-transitory storage medium, wherein, when the instructions are executed, the one or more processors are configured to:
determine a data acquisition mode of each phase on the basis of a real-time motion signal of an object;
acquire original data of the object according to the data acquisition mode for the each phase;
classify the original data to obtain data of multiple categories, wherein data of each category corresponds to a different phase; and
performing image reconstruction on the data of multiple categories to obtain the four-dimensional magnetic resonance image.

20. A non-transitory computer-readable medium storing computer instructions, when a computer reads the computer instructions, the computer executes the follow method:
determining a data acquisition mode of each phase on the basis of a real-time motion signal of an object;
acquiring original data of the object according to the data acquisition mode;
classifying the original data to obtain data of multiple categories, wherein data of each category corresponds to a different phase; and
performing image reconstruction on the data of multiple categories to obtain a four-dimensional magnetic resonance image.
